# EUROPEAN PATENT APPLICATION

(11) **EP 4 678 769 A1**
(43) Date of publication of application: **14.01.2026**
(21) Application number: 24766511.0
(22) Date of filing: 07.03.2024
(51) Int. Cl.: C12Q 1/6886, C12Q 1/6851, C12N 15/11

(54) **USE OF PLASMA CELL-FREE DNA METHYLATION MARKER IN LIVER TUMOR DETECTION**

(30) Priority: 09.03.2023 CN 202310226723; 28.09.2023 CN 202311274876
(71) Applicant: Singlera Health Technologies (Shanghai) Ltd., Shanghai 201203 (CN); Singlera Genomics (China) Ltd., Yangzhou, Jiangsu 225127 (CN); Singlera Genomics (Jiangsu) Ltd., Yangzhou, Jiangsu 225127 (CN)
(72) Inventor: LIU, Rui, Shanghai 201318 (CN); WANG, Hui, Shanghai 201318 (CN); SU, Zhixi, Shanghai 201318 (CN); ZHOU, Shuang, Shanghai 201318 (CN); YANG, Qichang, Shanghai 201318 (CN); MA, Chengcheng, Shanghai 201318 (CN); XIE, Kehui, Shanghai 201318 (CN)
(74) Representative: Huang, Liwei
(86) International application number: PCT/CN2024/080559
(87) International publication number: WO 2024/183797

(57) **Abstract**

A use of a plasma cell-free DNA methylation marker in liver tumor detection is provided. Specifically, a kit for liver tumor detection is provided. The kit comprises a reagent for detecting the methylation level of a DNA methylation marker combination, wherein the DNA methylation marker combination is one or more selected from IKZF1, Septin9, IRF4, DAB2IP, TSPYL5, CHFR, BEND4, GRASP, GPAM, and BDH1. A model constructed by means of the methylation detection result of the marker combination can be used for early screening and recurrence monitoring of HCC.

## Description

### CROSS REFERENCE TO THE RELATED APPLICATIONS

This application claims the benefit of the priority of a Chinese Patent Application No. 202310226723.2 filed with the China National Patent Office on March 9, 2023 and entitled "USE OF PLASMA CELL-FREE DNA METHYLATION MARKER IN LIVER TUMOR DETECTION", and a Chinese Patent Application No. 202311274876.0 filed with the China National Patent Office on September 28, 2023 and entitled "DNA METHYLATION MARKER GENE COMBINATION, REAGENT AND APPLICATION FOR DETECTING LIVER TUMOR-RELATED DISEASES", the contents of both of which are incorporated herein by reference in their entirety.

### TECHNICAL FIELD

The present application belongs to the field of biotechnology, and in particular relates to a use of a plasma cell-free DNA methylation marker in liver tumor detection and a DNA methylation marker gene combination, a reagent and an application for detecting liver tumor-related diseases.

### BACKGROUND

Early detection of hepatocellular carcinoma can increase the chance of surgery and improve the survival rate. Currently, the early screening techniques that are widely used include alpha-fetoprotein (AFP) detection and ultrasound (US). However, their low sensitivity limits the technical screening and monitoring of the disease.

At present, abnormal DNA methylation in blood has been widely recognized as a non-invasive marker for cancer detection. It has been found in various cancers that alterations in DNA methylation lead to abnormal gene expression, which is a critical event in the early stage of carcinogenesis. DNA methylation in hepatocellular carcinoma can reliably distinguish cancer patients from healthy individuals. However, an extremely small amount of abnormally methylated DNA (ctDNA) is released from tumor cells into the peripheral blood in early-stage cancers, posing a technical challenge.

The rapid development of technologies for selective specific enrichment and amplification of methylated sequences has facilitated highly sensitive detection. The high-throughput next-generation sequencing (NGS) technology has been widely applied and has shown feasibility in the detection of methylation markers in gastrointestinal and hepatobiliary tumors. However, the complexity and high cost of NGS detection limit its clinical application.

Therefore, there is an urgent need to develop a liver tumor methylation screening technology that is highly accurate, widely applicable in clinical practice, low-cost, and simple and feasible.

### SUMMARY

Through extensive experiments and repeated explorations, the inventors of this application have unexpectedly discovered that some free DNA methylation markers in plasma can be used for liver tumor detection. Based on such a discovery, the present application provides a kit and a method for liver tumor detection.

In a first aspect, the present application provides a kit for liver tumor detection. The kit includes a reagent for detecting the methylation level of a DNA methylation marker combination. The DNA methylation marker combination is one or more selected from IKZF1, Septin9, IRF4, DAB2IP, TSPYL5, CHFR, BEND4, GRASP, GPAM, and BDH1.

In some embodiments, the DNA methylation markers are located on a genome at positions as below:
IKZF1, chr7: 50343720-50472799;
Septin9, chr17: 75276651-75496678;
IRF4, chr6: 391739-411447;
DAB2IP, chr9: 124329336-124547809;
TSPYL5, chr8: 98285717-98290176;
CHFR, chr12: 133398773-133532890;
BEND4, chr4: 42112955-42154895;
GRASP, chr12: 52400724-52409673;
GPAM, chr10: 113909624-113975135;
BDH1 region, chr3: 197236654-197300194.

In some embodiments, the combination of methylation markers includes:
IKZF1, chr7: 50343867-50343961;
Septin9, chr17: 75369558-75369622;
IRF4, chr6: 392282-392377;
DAB2IP, chr9: 124461322-124461391;
TSPYL5, chr8: 98290035-98290215;
CHFR, chr12: 133485000-133485067;
BEND4, chr4: 42153816-42153921;
GRASP, chr12: 52401083-52401169;
GPAM, chr10: 113943540: 113943739;
BDH1 region 1, chr3: 197281790: 197281989;
BDH1 region 2, chr3: 197282545: 197282744.

In some embodiments, the kit employs a method selected from PCR amplification, methylation microarray, liquid-phase microarray, bisulfite sequencing, first-generation sequencing, next-generation sequencing, and/or third-generation sequencing for detection.

In some embodiments, the PCR amplification is digital PCR or fluorescence quantitative PCR.

In some embodiments, the liver tumor is HCC.

In some embodiments, the detection includes early screening and recurrence monitoring of HCC.

In some embodiments, the detection includes auxiliary diagnosis of HCC.

In some embodiments, the reagent for detecting the methylation level of the DNA methylation marker combination includes a primer and/or a probe for quantitative DNA methylation-specific PCR (qMSP).

In some embodiments, the primer and/or the probe are as shown in Table 2 of the specification.

In a second aspect, the present application provides a method for screening a methylation marker for liver tumor detection, which includes the following steps:
defining consistent common methylation marker regions in an HCC tumor using a whole-genome methylation dataset; and
further optimizing and selecting based on the performance of distinguishing HCC from a control group in these regions by using methylation-specific PCR.

In a third aspect, the present application provides a combination of methylation markers obtained by screening using the screening method as described in the second aspect of the present application.

In some embodiments, the marker combination is one or more selected from IKZF1, Septin9, IRF4, DAB2IP, TSPYL5, CHFR, BEND4, GRASP, GPAM, and BDH1.

In one embodiment, the position information of the DNA methylation markers on the genome is as follows:
IKZF1, chr7: 50343720-50472799;
Septin9, chr17: 75276651-75496678;
IRF4, chr6: 391739-411447;
DAB2IP, chr9: 124329336-124547809;
TSPYL5, chr8: 98285717-98290176;
CHFR, chr12: 133398773-133532890;
BEND4, chr4: 42112955-42154895;
GRASP, chr12: 52400724-52409673;
GPAM, chr10: 113909624-113975135;
BDH1 region, chr3: 197236654-197300194.

In one embodiment, the combination of methylation markers includes:
IKZF1, chr7: 50343867-50343961;
Septin9, chr17: 75369558-75369622;
IRF4, chr6: 392282-392377;
DAB2IP, chr9: 124461322-124461391;
TSPYL5, chr8: 98290035-98290215;
CHFR, chr12: 133485000-133485067;
BEND4, chr4: 42153816-42153921;
GRASP, chr12: 52401083-52401169;
GPAM, chr10: 113943540: 113943739;
BDH1 region 1, chr3: 197281790: 197281989;
BDH1 region 2, chr3: 197282545: 197282744.

In a fourth aspect, the present application provides a method for constructing a liver tumor detection model, which includes the following steps:
a) nucleic acid extraction from a plasma sample: extracting cell-free DNA (cfDNA);
b) treatment of nucleic acids with bisulfite to transform unmethylated cytosine to uracil while maintaining the sequence of methylated cytosine unchanged;
c) pre-amplification and dilution of a target site;
d) fluorescence PCR detection;
e) constructing a model by applying a feature selection method of incremental feature selection (IFS) of a machine learning model to the Ct or delta Ct values obtained from the detection results.

In some embodiments, a threshold suitable for clinical use is selected based on the output values of the model to determine a positive result.

In some embodiments, the target site in the step c) is as shown in the third aspect of the present application.

In some embodiments, the primer and the probe for the fluorescence PCR detection in the step d) are as shown in Table 2 of the specification.

In some embodiments, the machine learning model in the step e) is selected from SVR model, random forest, logistic regression, and interaction tree.

To further improve the efficiency, sensitivity, and specificity of detecting the isolated DNA methylation for liver tumor-related diseases, improve the early screening and diagnosis efficiency of liver tumor-related diseases, reduce the clinical diagnosis and treatment burden, and improve the clinical application value, the present application further optimizes the screened DNA methylation markers through experiments, and provides a DNA methylation marker gene combination for detecting liver tumor-related diseases based on the optimization results.

In a fifth aspect, the present application provides a DNA methylation marker gene combination for detecting liver tumor-related diseases. The gene combination is selected from DNA sequences within at least any one subregion of at least two of the following genes:
DAB2IP gene, wherein the detectable subregion is:
   chr9: 124460896-124462275;
   chr9: 124461322-124461391;
   chr9: 124461043-124461118;
   chr9: 124360386-124362685;
   chr9: 124360868-124360966;
CHFR gene, wherein the detectable subregion is:
   chr12: 133483901-133485740;
   chr12: 133485000-133485067;
   chr12: 133484896-133484985;
   chr12: 133463431-133464810;
   chr12: 133464246-133464335;
   chr12: 133404551-133406390;
   chr12: 133405064-133405171;
   chr12: 133532431-133532890;
   chr12: 133532689-133532802;
GRASP gene, wherein the detectable subregion is:
   chr12: 52400724-52401698;
   chr12: 52401083-52401169;
   chr12: 52400965-52401055;
   chr12: 52406880-52409127;
   chr12: 52408471-52408566.

In one embodiment, the gene combination is any one selected from the following gene subregion combinations:
chr9: 124460896-124462275 and chr12: 133483901-133485740;
chr9: 124460896-124462275 and chr12: 52400724-52401698;
chr12: 133483901-133485740 and chr12: 52400724-52401698; and
chr9: 124460896-124462275, chr12: 133483901-133485740 and chr12: 52400724-52401698.

In one embodiment, the gene combination includes:
DAB2IP: chr9: 124460896-124462275, CHFR: chr12: 133483901-133485740, and GRASP: chr12: 52400724-52401698.

It can be understood that, in one embodiment, the method for detecting the gene combination provided by the present application includes specifically amplifying the DNA sequences of all genes in the gene combination, and determining and/or evaluating liver tumor-related diseases based on their DNA methylation status. Where, the specifically amplified region in the gene subregion can be selected arbitrarily.

In a sixth aspect, the present application provides a reagent for detecting the presence and/or level of DNA methylation status of all genes in the gene combination in a test sample isolated from an organism.

In one embodiment, the reagent includes a forward primer, a reverse primer and/or a probe for detecting all genes in the gene combination.

It can be understood that the forward primer, the reverse primer and/or the probe in the reagent provided by the present application for detecting all genes in the gene combination can be primers and probes suitable for use in fluorescence quantitative PCR, as long as they can amplify the target gene and enable quantitative detection.

In one embodiment, the test sample isolated from an organism includes plasma, tissue or cells, preferably plasma.

In another aspect, the present application provides an application of the gene combination and/or the reagent in preparation of a diagnostic product for liver tumor.

In one embodiment, the liver tumor-related diseases include hepatocellular carcinoma, hepatitis B, liver cirrhosis, hepatic cyst, and fatty liver.

In one embodiment, the diagnostic product for liver tumor-related diseases is used to confirm the presence of liver tumor-related diseases, evaluate the formation risk of liver tumor-related diseases, and/or evaluate the progression of liver tumor-related diseases.

In one embodiment, the diagnostic product includes a kit.

It can be understood that, in actual use, the reagent or kit or diagnostic product provided by the present application can be used to detect the presence and/or level of DNA methylation status of all genes in the gene combination in a test sample isolated from an organism, and confirm the presence of liver tumor-related diseases, evaluate the formation or formation risk of liver tumor-related diseases and/or evaluate the progression of liver tumor-related diseases based on the detection results.

In a seventh aspect, the present application provides a kit containing the reagent.

In one embodiment, the kit further includes a reagent for a PCR reaction system and/or a bisulfite conversion reagent.

In one embodiment, the reagent for the PCR reaction system includes a reagent necessary for and/or commonly used in PCR amplification.

In one embodiment, the bisulfite conversion reagent may be a commercially available kit.

The beneficial effects of the present application at least include:

The present application has developed a technology based on blood multi-gene qMSP (quantitative DNA methylation-specific PCR) for early screening and recurrence monitoring of HCC.

The high-performance marker combination identified by the present application shows good clinical application value in multi-center studies.

The present application further optimizes the gene subregions of liver tumor-related diseases and provides a subregion combination with better effects. The gene combination provided by the present application shows better sensitivity and specificity when used as a biomarker for the detection of plasma-isolated DNA methylation, significantly improving the accuracy of early screening or re-screening of liver tumor-related diseases and thus being beneficial to improving the screening efficiency.

### BRIEF DESCRIPTION OF THE DRAWINGS

To better describe and illustrate the embodiments and/or examples of the inventions disclosed herein, reference may be made to one or more attached drawings. The additional description or examples provided for the attached drawings should not be construed as limiting the scope of any one of the disclosed inventions, the embodiments and/or examples presently described, or the best mode presently understood for carrying out the inventions.
Fig. 1 shows the AUC of the marker combination SVR model in the training set and validation set of the test sample according to an embodiment of the present application.
Fig. 2 shows the sensitivity comparison of the marker combination SVR model and AFP for hepatocellular carcinoma according to an embodiment of the present application.
Fig. 3 shows that the marker combination SVR model has good detection efficacy for AFP-negative populations according to an embodiment of the present application.

### DETAILED DESCRIPTION OF THE EMBODIMENTS

To explain the overall concept of this application more clearly, the following detailed description is provided by way of examples in conjunction with the attached drawings of the specification.

Many specific details are set forth in the following description to facilitate a thorough understanding of the present application. However, the present application may also be implemented in other ways different from those described herein. Therefore, the scope of protection of the present application is not limited to the specific embodiments disclosed below.

In the description of the present specification, the descriptions referring to terms such as "one embodiment", "some embodiments", "example", "specific example", or "some examples" etc. mean that the specific features, structures, materials or characteristics described in connection with the embodiment or example are included in at least one embodiment or example of the present application. In the present specification, the illustrative representations of the above terms do not necessarily refer to the same embodiment or example. Moreover, the specific features, structures, materials or characteristics described may be combined in a suitable manner in any one or more embodiments or examples.

Unless otherwise defined, all technical terms used hereinafter have the same meaning as commonly understood by those skilled in the art. The professional terms used herein are only for the purpose of describing specific embodiments and are not intended to limit the scope of protection of the present application.

Unless otherwise specifically stated, various raw materials, reagents, instruments, equipment, etc. used in the present application are commercially available or can be prepared by existing methods.

### Example 1

### Experimental method:

Using RRBS sequencing data derived from hepatocellular carcinoma tissues, pericarcinous tissues, plasma and blood cell DNA of control populations, together with the methylation microarray data obtained from the public database TCGA, three candidate marker sets were generated: a marker set obtained by comparing hepatocellular carcinoma tissues with normal liver tissues in the RRBS data, a marker set obtained by comparing hepatocellular carcinoma tissues with healthy plasma in the RRBS data, and a marker set obtained by comparing hepatocellular carcinoma tissues with normal liver tissues in the methylation microarray. The final candidate markers are the intersection of the three candidate methylation markers filtered by the median of RRBS blood cell data. After these markers were tested by fluorescence PCR, a total of 11 methylation candidate markers (Table 1) were identified for the detection of hepatocellular carcinoma.

In plasma samples, the candidate markers were validated, and the test samples included those from a control group, a hepatocellular carcinoma group, and populations with interference such as hepatitis B and liver cirrhosis. The reference levels of the markers listed in Table 1 and the performance of the marker combinations were analyzed. Due to the limited amount of plasma cell-free DNA in individual samples, the target sites were pre-amplified prior to the fluorescence PCR detection. An SVR model was established in the training set, and the stability of the model was validated using the validation set. The steps include:
1) Nucleic acid extraction from a plasma sample. Cell-free DNA (cfDNA) was extracted by using the QIAamp Circulating Nucleic Acid kit (Qiagen, 55114) according to the operating steps provided by the manufacturer.
2) Treatment of nucleic acids with bisulfite to transform unmethylated cytosine to uracil while maintaining the sequence of methylated cytosine unchanged. The EZ DNA Methylation-Lightning Kit (Zymo research, D5031) was used for bisulfite conversion.
3) Pre-amplification and dilution of a target site. Using the primers listed in Table 2 on the ProFlexTM PCR System (Thermo Fisher) platform, the target DNA was first amplified with the primer pool at 95°C for 3 minutes, followed by 8 cycles of 95°C for 30 seconds and 56°C for 60 seconds. The amplified products were diluted 10-fold.
4) Fluorescence PCR detection using the primers and probes listed in Table 2. The amplified products were detected by quantitative PCR following the standard procedures (Novo Start Methy Light qPCR Super Mix (Novo Protein)) in the ABI 7500 real-time PCR thermal cycler.
5) Construction of a model by applying a feature selection method of incremental feature selection (IFS) based on the SVR (python v3.9.12, scikit-learn v1.1.2) model to the Ct values obtained from the detection results of the training set. An interpreting model for hepatocellular carcinoma risk was constructed and a threshold for positive determination was defined. This detection workflow was designated as HepaAiQ.
6) The training set data included qualified patients from multiple centers, including 293 cases of HCC, 96 cases of chronic hepatitis B (CHB) or liver cirrhosis, 23 cases of benign hepatic lesions (BHL), and 147 healthy controls (HC). It is planned to include more patients with early-stage disease (77% at stage 0/A), smaller tumor volume (median of 3.5 cm), and compensated liver function (81% Child-Pugh grade A). The validation set included 523 individuals, including 205 cases of hepatocellular carcinoma, 100 cases of hepatitis B/liver cirrhosis, 102 cases of benign hepatic space-occupying lesions, and 116 healthy controls.

**Table 1. 11 marker genes, gene coordinates and amplification region coordinates**

| Gene | Gene Hg19 Pos. | PCR region |
|---|---|---|
| IKZF1 | chr7: 50343720-50472799 | chr7: 50343867-50343961 |
| Septin9 | chr17: 75276651-75496678 | chr17: 75369558-75369622 |
| IRF4 | chr6: 391739-411447 | chr6: 392282-392377 |
| DAB2IP | chr9: 124329336-124547809 | chr9: 124461322-124461391 |
| TSPYL5 | chr8: 98285717-98290176 | chr8: 98290035-98290215 |
| CHFR | chr12: 133398773-133532890 | chr12: 133485000-133485067 |
| BEND4 | chr4: 42112955-42154895 | chr4: 42153816-42153921 |
| GRASP | chr12: 52400724-52409673 | chr12: 52401083-52401169 |
| GPAM | chr10: 113909624-113975135 | chr10: 113943540: 113943739 |
| BDH1 region 1 | chr3: 197236654-197300194 | chr3: 197281790: 197281989 |
| BDHI region 2 | chr3: 197236654-197300194 | chr3: 197282545: 197282744 |

**Table 2. Sequences of primers and probes**

| Gene | F primer | R primer | Probe |
|---|---|---|---|
| IKZF 1 | GACGACGTATTTT TTTCGTGTTTC (SEQ ID NO: 1) | GCGCACCTCTCGAC CG (SEQ ID NO: 2) | TTTGTATCGGAGTAGC GATTCGGGAGG (SEQ ID NO: 3) |
| Septin9 | GTAGTTGGATGG GATTATTT (SEQ ID NO: 4) | CACCCGCAAAATCC TCT (SEQ ID NO: 5) | TTGTTGCGGTCGCGGA CG (SEQ ID NO: 6) |
| IRF4 | AAAAAAAAAAA ACTCCACATTT (SEQ ID NO: 7) | TAGTTGNGGAGTTT GGG (SEQ ID NO: 8) | ATCGTACGTAAGGTTC GGAGCGA (SEQ ID NO: 9) |
| DAB2IP | CGtCGtACGTCGttC Gt (SEQ ID NO: 10) | GaTCGaCGCGaCTCGa C (SEQ ID NO: 11) | CGtTGTTGtTTGGCGAC GtCG (SEQ ID NO: 12) |
| TSPYL5 | GCGCGGGAGGAtT TTCG (SEQ ID NO: 13) | GCATCACGTCCTCTC GCG (SEQ ID NO: 14) | CGAtTTCGGtCGtTtATG GT (SEQ ID NO: 15) |
| CHFR | GCGTTTTAGTTAG ATAGGGCGG (SEQ ID NO: 16) | GAaCCCCTTCCCCGA AaC (SEQ ID NO: 17) | GCGGCGCGtAGTtTTAG GC (SEQ ID NO: 18) |
| BEND4 | TtTCGAAGTTTtCG GGTGCG (SEQ ID NO: 19) | CGaaaaCCGCCGACAC TTA (SEQ ID NO: 20) | TGCGtCGAtATtCGAtAC G (SEO ID NO: 21) |
| GRASP | GTGTACGCGGCG TGGAGG (SEQ ID NO: 22) | ACGCACCTTTCGaCG aaaCA (SEQ ID NO: 23) | TAtCGCGCGtTCGtCGTG Tt (SEQ ID NO: 24) |
| GPAM | gGtTGcgAGttTtAG GG (SEQ ID NO: 25) | CAcgCTCACACACTA TC (SEQ ID NO: 26) | CGTGAGCGGATTCGAG GGT (SEQ ID NO: 27) |
| BDHI region 1 | GGGGGtTcgGttAG AG (SEQ ID NO: 28) | aaACTaaCCAaaaTaaaA aTCCA (SEQ ID NO: 29) | TTGTTTTCGCGCGGGT G (SEQ ID NO: 30) |
| BDHI region 2 | TAGAGGGCgttAG GGAA (SEQ ID NO: 31) | CCCCCTaCCCATTTaa TTT (SEQ ID NO: 32) | TCGTTCGAGTTGTAGA GACGC (SEQ ID NO: 33) |

### Example 2

### Experimental results:

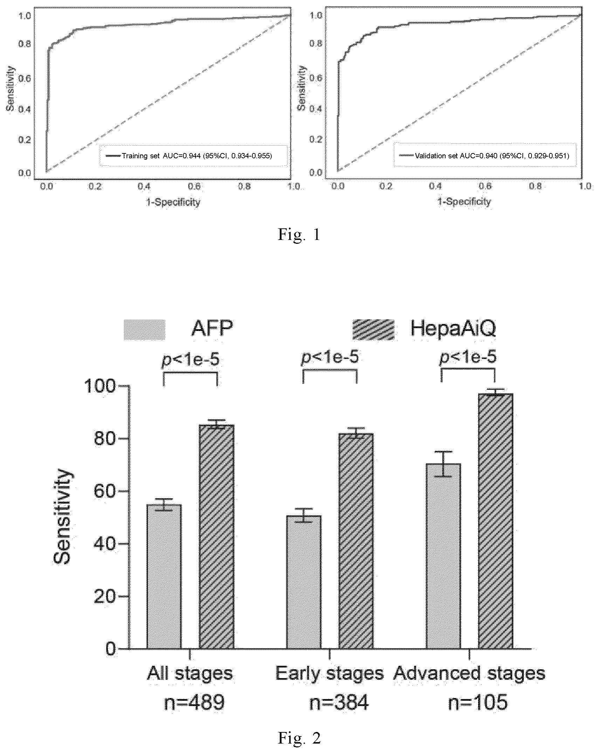

The results showed that the model established by the marker combination combined with SVR achieved good detection performance in both the training set and the validation set, with an AUC of 0.944 in the training set and 0.940 in the validation set (Fig. 1).

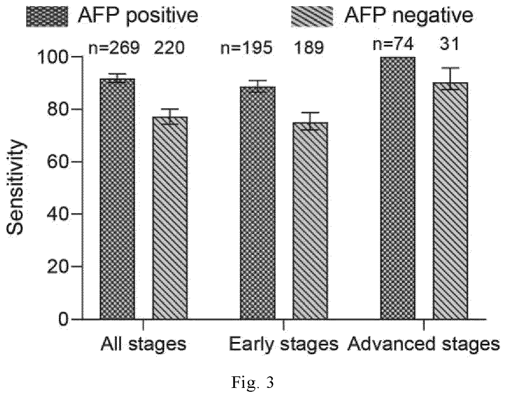

The HepaAiQ model was compared with AFP, a well-known commonly used clinical marker for HCC. In 489 HCC samples where both detection results were available (384 early-stage cases and 105 advanced-stage cases), the detection efficacy of the HepaAiQ detection model was significantly improved compared with that of the AFP detection, with significantly improved detection sensitivity for both early-stage and advanced-stage hepatocellular carcinoma, as shown in Fig. 2. In the AFP-negative population (269 AFP-positive patients and 220 AFP-negative patients), the HepaAiQ detection model also showed favorable detection performance (Fig. 3). The HepaAiQ detection model of the present application will fill the corresponding clinical gaps based on AFP.

### Example 3

### Experimental method:

Using RRBS sequencing data derived from hepatocellular carcinoma tissues, pericarcinous tissues, plasma and blood cell DNA of control populations, together with the methylation microarray data obtained from the public database TCGA, candidate marker sets were generated: a marker set obtained by comparing hepatocellular carcinoma tissues with normal liver tissues in the RRBS data, a marker set obtained by comparing hepatocellular carcinoma tissues with healthy plasma in the RRBS data, and a marker set obtained by comparing hepatocellular carcinoma tissues with normal liver tissues in the methylation microarray. After these markers were tested by fluorescence PCR, the methylation candidate markers of 11 target regions of three representative genes for hepatocellular carcinoma detection are shown in Table 3.

**Table 3 Position information of 11 target regions of three genes**

| Gene Name | Gene region hg19 | Subregion | PCR region | Amplification region No. |
|---|---|---|---|---|
| DAB2IP | chr9: 124329336-124547809 | chr9: 124460896-124462275 | Chr9: 124461322-124461391 | DAB2IP-1 |
| | | | Chr9: 124461525-124461614 | DAB2IP-2 |
| | | | Chr9: 124461417-124461522 | DAB2IP-3 |
| | | | Chr9: 124461681-124461785 | DAB2IP-4 |
| | | | Chr9: 124461797-124461893 | DAB2IP-5 |
| CHFR | chr12: 133398773-133532890 | chr12: 133483901-133485740 | Chr12: 133485000-133485067 | CHFR-1 |
| | | | Chr12: 133485097-133485195 | CHFR-2 |
| | | | Chr12: 133484905-133484998 | CHFR-3 |
| | | | Chr12: 133485314-133485397 | CHFR-4 |
| GRASP | chr12: 52400724-52409673 | chr12: 52400724-52401698 | Chr12: 52401083-52401169 | GRASP-1 |
| | | | Chr12: 52400965-52401055 | GRASP-2 |

In plasma samples, the candidate markers were validated, and the test samples included those from a control group, a hepatocellular carcinoma group, and populations with interference such as hepatitis B and liver cirrhosis. The reference levels of the markers listed in Table 3 and the performance of the marker combinations were analyzed. Due to the limited amount of plasma cell-free DNA in individual samples, the target regions were pre-amplified prior to the fluorescence PCR detection. A binary logistic regression model was established in the training set, and the stability of the model was validated using the test set. The steps include:
(1) Nucleic acid extraction from a plasma sample. Cell-free DNA (cfDNA) was extracted by using the QIAamp Circulating Nucleic Acid kit (Qiagen, 55114) according to the operating steps provided by the manufacturer.
(2) Treatment of nucleic acids with bisulfite to transform unmethylated cytosine to uracil while maintaining the sequence of methylated cytosine unchanged. The EZ DNA Methylation-Lightning Kit (Zymo research, D5031) was used for bisulfite conversion.
(3) Pre-amplification and dilution of a target region. Using the primers listed in Table 4 on the ProFlexTM PCR System (Thermo Fisher) platform, the target DNA was first amplified with the primer pool at 95°C for 3 minutes, followed by 8 cycles of 95°C for 30 seconds and 56°C for 60 seconds. The amplified products were diluted 10-fold.
(4) Fluorescence PCR detection using the primers and probes listed in Table 4. The amplified products were detected by quantitative PCR following the standard procedures (Novo Start Methy Light qPCR Super Mix (Novo Protein)) in the ABI 7500 real-time PCR thermal cycler.
(5) Construction of a model by using the Ct values obtained from the detection results of the training set based on binary logistic regression, including a two-target region combination model and a three-target region combination model. Receiver operating characteristic (ROC) curves were plotted to evaluate the ability of marker regions and marker combinations to discriminate between hepatocellular carcinoma patients and non-hepatocellular carcinoma patients.
(6) The training set, with a total of 467 individuals, included 233 cases of hepatocellular carcinoma (78.5% at BCLC stage 0/A), 93 cases of chronic hepatitis B (CHB) or liver cirrhosis, 18 cases of hepatic cyst or fatty liver, and 123 healthy controls (HC) from multiple centers. The test set included 466 individuals, including 209 cases of hepatocellular carcinoma (75.6% at BCLC stage 0/A), 92 cases of hepatitis B/liver cirrhosis, 25 cases of hepatic cyst or fatty liver, and 140 healthy controls (HC).

**Table 4 Primers & Probes**

| Amplification region No. | Upstream primer | Downstream primer | Probe |
|---|---|---|---|
| DAB2IP-1 | CGttCGttACGTCGtttt CGt (SEQ ID NO: 34) | GaTCGaCGCGaCTCGa C (SEQ ID NO: 35) | CGtTGTTGttTTGGCG ACGtCG (SEQ ID NO: 36) |
| DAB2IP-2 | tTGAGGAAGtttTGcGt AcGG (SEQ ID NO: 37) | CgaaTCCCgCgCgaaT (SEQ ID NO: 38) | GGGGTTAGcGcGGcG G (SEQ ID NO: 39) |
| DAB2IP-3 | tttTTGtTGGGcGGtcGc (SEQ ID NO: 40) | aCCgaaCCgTCgaCgCTC g (SEQ ID NO: 41) | cGGtcGAGGGcGGGtc G (SEQ ID NO: 42) |
| DAB2IP-4 | tAGttcGGGGcGGcG (SEQ ID NO: 43) | CCgCCACATCCTaCCg (SEQ ID NO: 44) | TGTtcGcGGcGGcGG (SEQ ID NO: 45) |
| DAB2IP-5 | cGGtcGAGGTGGttttA TAAGT (SEQ ID NO: 46) | ACgCgaaaACAAAaCgC g (SEQ ID NO: 47) | TGTGGtTcGtttcGGGtc GG (SEQ ID NO: 48) |
| CHFR-1 | GCGTTTTAGTTAGA TAGGGCGG (SEQ ID NO: 49) | GAaaaCCCCTTCCCCG AAaC (SEQ ID NO: 50) | GCGGCGCGtAGTtTT AGGC (SEQ ID NO: 51) |
| CHFR-2 | GAtAcGTTGGcGAAT TttttcGtt (SEQ ID NO: 52) | CgaaaCgaAaAaCCgCgC (SEQ ID NO: 53) | AGAtAGtATGTTGttT AGtAGcGTGcGG (SEQ ID NO: 54) |
| CHFR-3 | cGTGGGAGGAGcGc G (SEQ ID NO: 55) | aaTCgaCCgCCCCACA (SEQ ID NO: 56) | GGcGAGAttcGGAGtc GcG (SEQ ID NO: 57) |
| CHFR-4 | GGGAAGAtAAAcGA cGtTtTGAA (SEQ ID NO: 58) | AACCCAaCAaCgCTaA CTaAaC (SEQ ID NO: 59) | TAcGTTtcGGtAtTTcGc GGcG (SEQ ID NO: 60) |
| GRASP-1 | GttttTtTtCgAtTtttTAtA GGG (SEQ ID NO: 61) | aaCcgaaaAAaAAaaTaaa aaACTC (SEQ ID NO: 62) | TGTTTTTTTTTCGGC GTTCGCG (SEQ ID NO: 63) |
| GRASP-2 | GtTGTACGCGGCGtT GGAGG (SEQ ID NO: 64) | ACGCACCTTTCGaCGa aaCA (SEQ ID NO: 65) | TAtCGCGCGtTCGtCG TGTt (SEQ ID NO: 66) |

### Experimental results:

ROC curve analysis was performed on the Ct values of 11 target regions of the three genes in both the training set and the test set, and the AUC values were calculated. All 11 target regions, whether in the training set or the test set, demonstrated the ability to discriminate between hepatocellular carcinoma and non-hepatocellular carcinoma. The specific results are shown in Table 5.

**Table 5 AUC values of various target regions in the training set and the test set**

| Amplification region No. | Training set AUC | Test set AUC |
|---|---|---|
| DAB2IP-1 | 0.734 | 0.729 |
| DAB2IP-2 | 0.724 | 0.726 |
| DAB2IP-3 | 0.704 | 0.701 |
| DAB2IP-4 | 0.701 | 0.708 |
| DAB2IP-5 | 0.706 | 0.716 |
| CHFR-1 | 0.734 | 0.726 |
| CHFR-2 | 0.727 | 0.711 |
| CHFR-3 | 0.705 | 0.714 |
| CHFR-4 | 0.701 | 0.715 |
| GRASP-1 | 0.731 | 0.727 |
| GRASP-2 | 0.70 | 0.713 |

A two-target region combination model and a three-target region combination model were constructed using the data from the training set. The AUC results of the representative models with excellent performance in the combination are shown in Table 6, and the AUC results of the representative models with excellent performance in the test set are shown in Table 7. The fact that the AUC values of the combination are all greater than those of individual target regions indicates that the combination of target regions can enhance the ability to discriminate between hepatocellular carcinoma and non-hepatocellular carcinoma. It should be noted that the AUC of the three-target region combination model is as high as 0.921 in the training set and 0.919 in the test set.

**Table 6 Performance of Representative Model Combinations in the Training Set**

| Model No. | Amplification region combination | AUC in Training set | Sensitivity in Training set | Specificity in Training set |
|---|---|---|---|---|
| Combination 1 | DAB2IP-1+ CHFR-1 | 0.829 | 70.0% | 90.8% |
| Combination 2 | DAB2IP-2+ CHFR-2 | 0.812 | 73.1% | 77.9% |
| Combination 3 | CHFR-1+ GRASP-1 | 0.867 | 80.6% | 86.3% |
| Combination 4 | CHFR-1+ GRASP-2 | 0.818 | 69.2% | 83.3% |
| Combination 5 | DAB2IP-3+ GRASP-2 | 0.810 | 64.3% | 88.3% |
| Combination 6 | DAB2IP-4+ GRASP-2 | 0.830 | 64.3% | 92.9% |
| Combination 7 | DAB2IP-1+CHFR-1+ GRASP-1 | 0.921 | 88.5% | 86.3% |
| Combination 8 | DAB2IP-1+CHFR-3+ GRASP-2 | 0.911 | 78.9% | 92.5% |

**Table 7 Performance of Representative Model Combinations in the Test set**

| Model No. | Amplification region combination | AUC in Test set | Sensitivity in Test set | Specificity in Test set |
|---|---|---|---|---|
| Combination 1 | DAB2IP-1+ CHFR-1 | 0.822 | 67.9% | 87.6% |
| Combination 2 | DAB2IP-2+ CHFR-2 | 0.810 | 76.7% | 76.1% |
| Combination 3 | CHFR-1+ GRASP-1 | 0.857 | 81.4% | 82.1% |
| Combination 4 | CHFR-1+ GRASP-2 | 0.806 | 66.5% | 82.1% |
| Combination 5 | DAB2IP-3+ GRASP-2 | 0.813 | 63.7% | 88.8% |
| Combination 6 | DAB2IP-4+ GRASP-2 | 0.814 | 60.9% | 90.4% |
| Combination 7 | DAB2IP-1+CHFR-1+ GRASP-1 | 0.919 | 84.7% | 88.1% |
| Combination 8 | DAB2IP-1+CHFR-3+ GRASP-2 | 0.901 | 79.7% | 92.4% |

### Example 4

### Experimental method:

In plasma samples, the markers were detected, and the test samples included those from a control group, a hepatocellular carcinoma group, and populations with interference such as hepatitis B and liver cirrhosis. The reference levels of the markers listed in Table 8 and the performance of the marker combinations were analyzed. Due to the limited amount of plasma cell-free DNA in individual samples, the target regions were pre-amplified prior to the fluorescence PCR detection. A binary logistic regression model was established in the training set, and the stability of the model was validated using the test set. The steps include:
(1) Nucleic acid extraction from a plasma sample. Cell-free DNA (cfDNA) was extracted by using the QIAamp Circulating Nucleic Acid kit (Qiagen, 55114) according to the operating steps provided by the manufacturer.
(2) Treatment of nucleic acids with bisulfite to transform unmethylated cytosine to uracil while maintaining the sequence of methylated cytosine unchanged. The EZ DNA Methylation-Lightning Kit (Zymo research, D5031) was used for bisulfite conversion.
(3) Pre-amplification and dilution of a target region. Using the primers listed in Table 9 on the ProFlexTM PCR System (Thermo Fisher) platform, the target DNA was first amplified with the primer pool at 95°C for 3 minutes, followed by 8 cycles of 95°C for 30 seconds and 56°C for 60 seconds. The amplified products were diluted 10-fold.
(4) The amplified products were detected by quantitative PCR following the standard procedures (Novo Start Methy Light qPCR Super Mix (Novo Protein)) in a cycler.
(5) Construction of a combination model by using the Ct values obtained from the detection results of the training set based on binary logistic regression. Receiver operating characteristic (ROC) curves were plotted to evaluate the ability of marker regions and marker combinations to discriminate between hepatocellular carcinoma patients and non-hepatocellular carcinoma patients.
(6) The training set, with a total of 467 individuals, included 233 cases of hepatocellular carcinoma (78.5% at BCLC stage 0/A), 93 cases of chronic hepatitis B (CHB) or liver cirrhosis, 18 cases of hepatic cyst or fatty liver, and 123 healthy controls (HC) from multiple centers. The test set included 466 individuals, including 209 cases of hepatocellular carcinoma (75.6% at BCLC stage 0/A), 92 cases of hepatitis B/liver cirrhosis, 25 cases of hepatic cyst or fatty liver, and 140 healthy controls (HC).

**Table 8 Position information of amplification genes and target regions**

| Gene Name | Gene region hg19 | Subregion | PCR region | Amplification region No. |
|---|---|---|---|---|
| DAB2IP | chr9: 124329336-124547809 | chr9: 124460896-124462275 | Chr9: 124461322-124461391 | DAB2IP-1 |
| CHFR | chr12: 133398773-133532890 | chr12: 133483901-133485740 | Chr12: 133485000-133485067 | CHFR-1 |
| GRASP | chr12: 52400724-52409673 | chr12: 52400724-52401698 | Chr12: 52401083-52401169 | GRASP-1 |
| BDH1 | chr3: 197236654-197300194 | Chr3: 197281606-197283793 | chr3: 197281790-197281989 | BDH1-1 |
| KCNA3 | chr1: 111214310-111217655 | chr1: 111217583: 111217895 | chr1: 111217718-111217817 | KCNA3-1 |

**Table 9 Primers & Probes**

| Amplification region No. | Upstream primer | Downstream primer | Probe |
|---|---|---|---|
| DAB2IP-1 | CGttCGttACGTCGttttCGt (SEQ ID NO: 34) | GaTCGaCGCGaCTCGa C (SEQ ID NO: 35) | CGtTGTTGttTTGGCGA CGtCG (SEQ ID NO: 36) |
| CHFR-1 | GCGTTTTAGTTAGATA GGGCGG (SEQ ID NO: 49) | GAaaaCCCCTTCCCC GAAaC (SEQ ID NO: 50) | GCGGCGCGtAGTtTTA GGC (SEQ ID NO: 51) |
| GRASP-1 | GttttTtTtCgAtTtttTAtAGG G (SEQ ID NO: 61) | aaCcgaaaAAaAAaaTaaa aaACTC (SEQ ID NO: 62) | TGTTTTTTTTCGGCG TTCGCG (SEQ ID NO: 63) |
| BDH1-1 | GGGGGTTCGGTTAGAG (SEQ ID NO: 67) | AAACTAACCAAAAT AAAAATCCA (SEQ ID NO: 68) | TTGTTTTCGCGCGGG TG (SEQ ID NO: 69) |
| KCNA3-1 | GttTGtATTTtTTGtAGtttTt AAG (SEQ ID NO: 70) | CcNaCTCTaCTaaaTaaa CATT (SEQ ID NO: 71) | TGTTGTATTTCGGCG TTTCGCG (SEQ ID NO: 72) |

### Experimental results:

A two-target region combination model and a three-target region combination model were constructed using the data from the training set. The AUC of the representative model combinations in the training set and the test set is shown in Table 10 and 11, respectively.

**Table 10 Performance of Representative Model Combinations in the Training Set**

| Model No. | Amplification region combination | AUC in Training set | Sensitivity in Training set | Specificity in Training set |
|---|---|---|---|---|
| Combination 1 | DAB2IP-1+ CHFR-1 | 0.829 | 70.0% | 90.8% |
| Combination 3 | CHFR-1+ GRASP-1 | 0.867 | 80.6% | 86.3% |
| Combination 9 | DAB2IP-1+ BDH1-1 | 0.743 | 54.5% | 89.7% |
| Combination 7 | DAB2IP-1 +CHFR-1 +GRASP-1 | 0.921 | 88.5% | 86.3% |
| Combination 10 | DAB2IP-1+GRASP-1+BDH1-1 | 0.829 | 63.1% | 89.3% |
| Combination 11 | DAB2IP-1+CHFR-1+KCNA3-1 | 0.816 | 61.0% | 88.9% |
| Combination 12 | CHFR-1+ GRASP-1+ KCNA3-1 | 0.822 | 61.9% | 88.0% |

**Table 11 Performance of Representative Model Combinations in the Test set**

| Model No. | Amplification region combination | AUC in Test set | Sensitivity in Test set | Specificity in Test set |
|---|---|---|---|---|
| Combination 1 | DAB2IP-1+ CHFR-1 | 0.822 | 67.9% | 87.6% |
| Combination 3 | CHFR-1+ GRASP-1 | 0.857 | 81.4% | 82.1% |
| Combination 9 | DAB2IP-1+ BDH1-1 | 0.735 | 54.1% | 86.9% |
| Combination 7 | DAB2IP-1 +CHFR-1 +GRASP-1 | 0.919 | 84.7% | 88.1% |
| Combination 10 | DAB2IP-1+GRASP-1+BDH1-1 | 0.824 | 62.2% | 87.2% |
| Combination 11 | DAB2IP-1+CHFR-1+KCNA3-1 | 0.820 | 63.2% | 89.9% |
| Combination 12 | CHFR-1+ GRASP-1+ KCNA3-1 | 0.819 | 63.6% | 86.4% |

### Example 5

### Experimental method:

The markers were detected in the tissue samples from 24 cases of hepatocellular carcinoma tissues and 24 cases of pericarcinous tissues. Specifically, the following steps are involved:

DNA was extracted from the tissues using the Qiagen QIAamp DNA FFPE Tissue Kit.

20 ng of the DNA samples obtained from the above step were treated with a bisulfite reagent (D5031, ZYMO RESEARCH) to obtain transformed DNA.

Optionally, using a primer pool containing the methylation-specific primers in Table X and forward and reverse primer pairs, PCR amplification was performed with the transformed DNA as the template, with each primer at a final concentration of 100 nM. The PCR reaction system contains 10 µL of the transformed DNA (including 2.5 µL of premix of the above primers) and 12.5 µL of PCR reagent (Luna^{®}Universal Probe qPCR Master Mix (NEB). The PCR reaction conditions are: 95°C for 5 minutes; followed by 10 cycles of 95°C for 30 seconds and 56°C for 60 seconds.

The obtained pre-amplification products were diluted 10-fold and subjected to multiplex fluorescence PCR detection to obtain the fluorescence Ct values of the markers. In the reaction system of fluorescence PCR, each primer has a final concentration of 500 nM, and each detection probe has a final concentration of 200 nM. The PCR reaction system contains: 10 µL of diluted pre-amplification products, 2.5 µL of a primer and probe premix containing the detection sites, and 12.5 µL of PCR reagent (Luna^{®}Universal Probe qPCR Master Mix (NEB)). The PCR reaction conditions are as follows: 95°C for 5 minutes, followed by 50 cycles of 95°C for 30 seconds and 56°C for 60 seconds (for collecting fluorescence). Different fluorescence was detected in the corresponding fluorescence channels using the ABI 7500 Real-Time PCR System. The Ct values of samples obtained from the buffy coat, pericarcinous tissues, and carcinoma tissues were calculated and compared. For targets with no detectable amplification signals, the Ct value was set to 50.

**Table 12 Position information of amplification genes and target regions**

| Gene Name | Gene region hg19 | Subregion | PCR region | Amplification region No. |
|---|---|---|---|---|
| DAB2IP | chr9: 124329336-124547809 | chr9: 124460896-124462275 | Chr9: 124461322-124461391 | DAB2IP-1 |
| | | chr9: 124360386-124362685 | chr9: 124360868-124360966 | DAB2IP-6 |
| CHFR | chr12: 133398773-133532890 | chr12: 133483901-133485740 | Chr12: 133485000-133485067 | CHFR-1 |
| | | chr12: 133463431-133464810 | chr12: 133464246-133464335 | CHFR-5 |
| GRASP | chr12: 52400724-52409673 | chr12: 52400724-52401698 | Chr12: 52401083-52401169 | GRASP-1 |
| | | chr12: 52406880-52409127 | Chr12: 52408471-52408566 | GRASP-3 |

**Table 13 Primers & Probes**

| Amplification region No. | Upstream primer | Downstream primer | Probe |
|---|---|---|---|
| DAB2IP-1 | CGttCGttACGTCGt tttCGt (SEQ ID NO: 34) | GaTCGaCGCGaCTCG aC (SEQ ID NO: 35) | CGtTGTTGttTTGGC GACGtCG (SEQ ID NO: 36) |
| CHFR-1 | GCGTTTTAGTTA GATAGGGCGG (SEQ ID NO: 49) | GAaaaCCCCTTCCCC GAAaC (SEQ ID NO: 50) | GCGGCGCGtAGTtT TAGGC (SEQ ID NO: 51) |
| GRASP-1 | GttttTtTtCgAtTtttT AtAGGG (SEQ ID NO: 61) | aaCcgaaaAAaAAaaTaa aaaACTC (SEQ ID NO: 62) | TGTTTTTTTTTCG GCGTTCGCG (SEQ ID NO: 63) |
| DAB2IP-6 | AGttTGCGCGtTtT GCGAGG (SEQ ID NO: 73) | AACAaaCCCCGaaCA AaTaCCC (SEQ ID NO: 74) | tAGGCGCGtttTCGttt TACG (SEQ ID NO: 75) |
| CHFR-5 | AGATGGtCGAGC GtttTttACG (SEQ ID NO: 76) | aaCTaaCGaCGaCGCT CACCA (SEQ ID NO: 77) | TCGCGGGttAtTttC GGtTtCG (SEQ ID NO: 78) |
| GRASP-3 | TGCGtAGGttTGGT GGTGAAG (SEQ ID NO: 79) | CAaCGAaCCCGaaAaC AaaCCC (SEQ ID NO: 80) | ACGAtACGtTGGA GTCGGTGC (SEQ ID NO: 81) |

### Experimental results:

The detection results were combined by logistic regression. The AUC results of the models with excellent performance in discriminating between pericarcinous tissues and carcinoma tissues among these combinations are shown in Table 14.

**Table 14 Performance of representative model combinations**

| Model No. | target regions combination | AUC |
|---|---|---|
| Combination 7 | DAB2IP-1 | 0.913 |
| | CHFR-1 | |
| | GRASP-1 | |
| Combination 13 | DAB2IP-6 | 0.738 |
| | CHFR-5 | |
| | GRASP-3 | |
| Combination 14 | DAB2IP- 1 | 0.833 |
| | CHFR- 1 | |
| | GRASP-3 | |

The foregoing has shown and described the fundamental principles, main features, and advantages of the present application. For those skilled in the art, it is evident that the present application is not limited to the details of the exemplary embodiments described above and may be embodied in other specific forms without departing from the spirit or essential characteristics of the present application. Therefore, in all perspectives, the embodiments should be regarded as exemplary and non-restrictive. The scope of the present application is defined by the appended claims, rather than by the foregoing description, and it is intended that all variations that fall within the meaning and scope of the equivalents of the claims are encompassed within the present application.

In addition, it can be understood that although the present specification is described in terms of embodiments, each embodiment does not necessarily include only a single independent technical solution. Such a manner of description in the specification is merely for the sake of clarity. Those skilled in the art should regard the specification as a whole, and the technical solutions described in the respective embodiments may also be appropriately combined to form other embodiments that can be understood by those skilled in the art.

The embodiments in the present specification are described in a progressive manner. For the identical or similar parts among the embodiments, they can be referred to each other, with each embodiment emphasizing the differences from the others. The above description is merely illustrative of the embodiments of the present application and is not intended to limit the present application. For those skilled in the art, various modifications and variations may be made to the present application. Any modifications, equivalent substitutions, or improvements made within the spirit and principles of the present application are intended to be included within the scope of the claims of the present application.

## Claims

1. A kit for liver tumor detection, comprising a reagent for detecting the methylation level of a DNA methylation marker combination, wherein the DNA methylation marker combination is one or more selected from IKZF1, Septin9, IRF4, DAB2IP, TSPYL5, CHFR, BEND4, GRASP, GPAM, and BDH1.

2. The kit according to claim 1, wherein the DNA methylation markers are located on a genome as below:
IKZF1, chr7: 50343720-50472799;
Septin9, chr17: 75276651-75496678;
IRF4, chr6: 391739-411447;
DAB2IP, chr9: 124329336-124547809;
TSPYL5, chr8: 98285717-98290176;
CHFR, chr12: 133398773-133532890;
BEND4, chr4: 42112955-42154895;
GRASP, chr12: 52400724-52409673;
GPAM, chr10: 113909624-113975135;
BDH1 region, chr3: 197236654-197300194.

3. The kit according to claim 2, wherein the methylation marker combination includes:
IKZF1, chr7: 50343867-50343961;
Septin9, chr17: 75369558-75369622;
IRF4, chr6: 392282-392377;
DAB2IP, chr9: 124461322-124461391;
TSPYL5, chr8: 98290035-98290215;
CHFR, chr12: 133485000-133485067;
BEND4, chr4: 42153816-42153921;
GRASP, chr12: 52401083-52401169;
GPAM, chr10: 113943540: 113943739;
BDH1 region 1, chr3: 197281790: 197281989;
BDH1 region 2, chr3: 197282545: 197282744.

4. The kit according to any one of claims 1-3, wherein the kit is used for a method selected from PCR amplification, methylation microarray, liquid-phase microarray, bisulfite sequencing, first-generation sequencing, next-generation sequencing, and/or third-generation sequencing for detection.

5. The kit according to claim 4, wherein the PCR amplification is digital PCR or fluorescence quantitative PCR.

6. The kit according to any one of claims 1-5, wherein the liver tumor is HCC.

7. The kit according to claim 6, wherein the detection comprises early screening and recurrence monitoring of HCC.

8. The kit according to claim 6, wherein the detection comprises auxiliary diagnosis of HCC.

9. The kit according to any one of claims 1-8, wherein the reagent for detecting the methylation level of the DNA methylation marker combination comprises a primer and/or a probe for quantitative DNA methylation-specific PCR (qMSP).

10. The kit according to claim 9, wherein the primer and/or the probe are as shown in Table 2 of the specification.

11. A method for screening a methylation marker for liver tumor detection, comprising the following steps:
defining consistent common methylation marker regions in an HCC tumor using a whole-genome methylation dataset; and
further optimizing and selecting based on the performance of distinguishing HCC from a control group in these regions by using methylation-specific PCR.

12. A combination of methylation markers obtained by screening using the screening method according to claim 11.

13. The combination of methylation markers according to claim 12, wherein the combination is selected from IKZF1, Septin9, IRF4, DAB2IP, TSPYL5, CHFR, BEND4, GRASP, GPAM, and BDH1.

14. The combination of methylation markers according to claim 13, wherein position information of the DNA methylation markers on the genome is as follows:
IKZF1, chr7: 50343720-50472799;
Septin9, chr17: 75276651-75496678;
IRF4, chr6: 391739-411447;
DAB2IP, chr9: 124329336-124547809;
TSPYL5, chr8: 98285717-98290176;
CHFR, chr12: 133398773-133532890;
BEND4, chr4: 42112955-42154895;
GRASP, chr12: 52400724-52409673;
BDH1 region, chr3: 197236654-197300194.

15. The combination of methylation markers according to claim 14, wherein the combination of methylation markers comprises:
IKZF1, chr7: 50343867-50343961;
Septin9, chr17: 75369558-75369622;
IRF4, chr6: 392282-392377;
DAB2IP, chr9: 124461322-124461391;
TSPYL5, chr8: 98290035-98290215;
CHFR, chr12: 133485000-133485067;
BEND4, chr4: 42153816-42153921;
GRASP, chr12: 52401083-52401169;
GPAM, chr10: 113943540: 113943739;
BDH1 region 1, chr3: 197281790: 197281989;
BDH1 region 2, chr3: 197282545: 197282744.

16. A method for constructing a liver tumor detection model, comprising the following steps:
a) nucleic acid extraction from a plasma sample: extracting cell-free DNA (cfDNA);
b) treating nucleic acids with bisulfite, so as to transform unmethylated cytosine to uracil, while maintaining the sequence of methylated cytosine unchanged;
c) pre-amplification of a target site, and dilution;
d) fluorescence PCR detection;
e) constructing a model, by applying a feature selection method of incremental feature selection (IFS) of a machine learning model to the Ct or delta Ct values obtained from the detection results, and selecting a threshold suitable for clinical use for positive determination, based on the output values of the model.

17. The constructing method according to claim 16, wherein the target site in the step c) is as shown in any one of claims 12-15.

18. The constructing method according to claim 16, wherein the primer and the probe for the fluorescence PCR detection in the step d) are as shown in Table 2 of the specification.

19. The constructing method according to claim 16, wherein the machine learning model in the step e) is selected from SVR model, random forest, logistic regression, and interaction tree.

20. A DNA methylation marker gene combination for use in detecting liver tumor-related diseases, wherein the gene combination comprises DNA sequences from at least two of genes as below, wherein each DNA sequence is located within at least any one subregion as below:
DAB2IP gene, wherein detectable subregions are:
chr9: 124460896-124462275;
chr9: 124461322-124461391;
chr9: 124461043-124461118;
chr9: 124360386-124362685;
chr9: 124360868-124360966;
CHFR gene, wherein detectable subregions are:
chr12: 133483901-133485740;
chr12: 133485000-133485067;
chr12: 133484896-133484985;
chr12: 133463431-133464810;
chr12: 133464246-133464335;
chr12: 133404551-133406390;
chr12: 133405064-133405171;
chr12: 133532431-133532890;
chr12: 133532689-133532802;
GRASP gene, wherein detectable subregion are:
chr12: 52400724-52401698;
chr12: 52401083-52401169;
chr12: 52400965-52401055;
chr12: 52406880-52409127;
chr12: 52408471-52408566.

21. The gene combination according to claim 20, wherein the gene combination is any one selected from the following gene subregion combinations:
chr9: 124460896-124462275 and chr12: 133483901-133485740;
chr9: 124460896-124462275 and chr12: 52400724-52401698;
chr12: 133483901-133485740 and chr12: 52400724-52401698;
chr9: 124460896-124462275, chr12: 133483901-133485740, and chr12: 52400724-52401698.

22. A reagent for detecting the presence and/or level of DNA methylation status of genes in the gene combination according to claim 20 or 21 in a test sample isolated from an organism.

23. The reagent according to claim 22, wherein the reagent comprises a forward primer, a reverse primer and/or a probe for detecting genes in the gene combination.

24. The reagent according to claim 23, wherein the test sample isolated from an organism comprises plasma, tissue, and cells.

25. Use of the gene combination according to claim 20 or 21 and/or the reagent according to any one of claims 22-24 in preparation of a diagnostic product for liver tumor-related diseases.

26. The use according to claim 25, wherein the liver tumor-related diseases comprise hepatocellular carcinoma, hepatitis B, liver cirrhosis, hepatic cyst, and fatty liver.

27. The application according to claim 25, wherein the diagnostic product for liver tumor-related diseases is used to confirm the presence of liver tumor-related diseases, evaluate the formation risk of liver tumor-related diseases, and/or evaluate the progression of liver tumor-related diseases.

28. A kit comprising the reagent according to any one of claims 22-24.

29. The kit according to claim 28, wherein the kit further comprises a reagent for a PCR reaction system and/or a bisulfite conversion reagent.
